# EUROPEAN PATENT APPLICATION

(11) **EP 2 022 789 A1**
(43) Date of publication of application: **11.02.2009**
(21) Application number: 07113888.7
(22) Date of filing: 06.08.2007
(51) Int. Cl.: C07D 401/12

(54) **Process for the preparation of a gastric acid secretion inhibitor**

(71) Applicant: Farmaprojects, S.A., 08902 L'Hospitalet de Llobregat (Barcelona) (ES)
(72) Inventor: Bessa Belmunt, Jordi, 08004, Barcelona (ES); García García, Elena, 08203, Sabadell (Barcelona) (ES); Riego Arboleya, Estela, 08980, Sant Feliu de Llobregat (Barcelon (ES)
(74) Representative: Barlocci, Anna

(57) **Abstract**

The present process comprises the preparation of rabeprazole or a pharmaceutically acceptable salt thereof by oxidation in an acidic media of a N-oxide sulfide derivative compound, followed by a further selective reduction of the N-oxide obtained to afford rabeprazole.

## Description

The present invention relates to a process for preparing rabeprazole.

### BACKGROUND OF THE INVENTION

Rabeprazole is a substituted benzimidazole proton-pump inhibitor of formula (I):

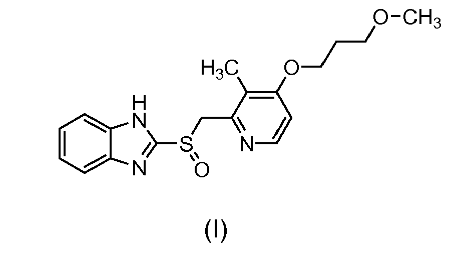

Rabeprazole suppresses gastric acid secretion by inhibiting the gastric H⁺/K⁺ATPase at the secretory surface of the gastric parietal cell. Because this enzyme is regarded as the acid (proton) pump within the parietal cell, rabeprazole has been characterized as a gastric proton-pump inhibitor. This product can be used for prevention and treatment of gastric-acid related diseases, including gastro-oesophageal reflux, gastritis, duodenitis, gastric ulcer and duodenal ulcer.

Rabeprazole is marketed in the sodium salt form, 2-[[[4-(3-methoxypropoxy) - 3-methyl-2-pyridinyl]-methyl]sulfinyl]-1*H*-benzimidazole sodium salt, and was disclosed for the first time in US patent 5045552-A.

Several processes for the preparation of substituted benzimidazoles having a pyridine moiety and being useful as proton-pump inhibitors have been disclosed in the art. The synthesis commonly followed (Scheme 1) is based on the use of N-oxide pyridines, at the very first steps of the synthesis, with the aim of activating the pyridine making the subsequent reaction at the pyridine ring easier. Then, there is a rearrangement with acetic anhydride to obtain a pyridine with an acetoxy methylene at the alpha position that is further hydrolyzed and transformed into chloride. The resulting chloromethylpyridine is coupled with a benzimidazole compound to obtain a sulfide compound, which is a precursor of rabeprazole.

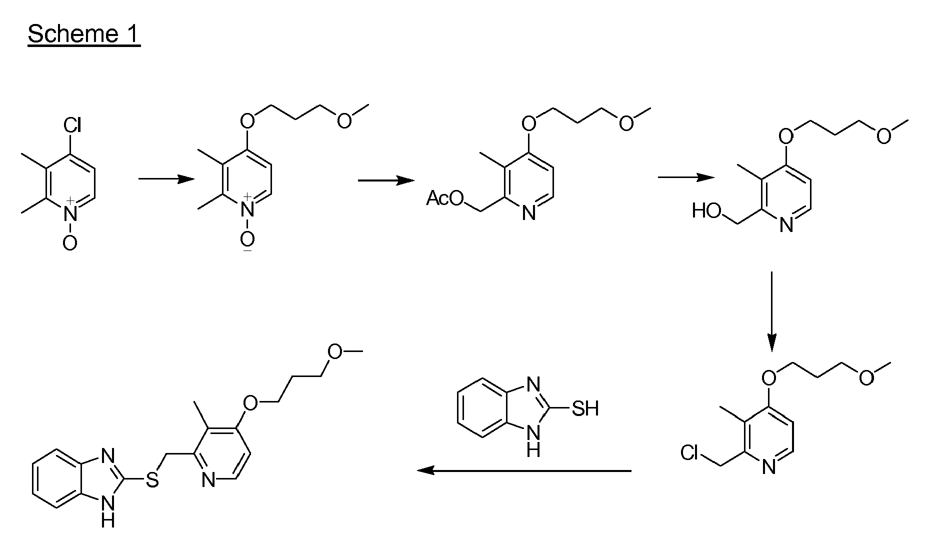

Thereafter as the last step of the process, the sulfide is oxidized to obtain the final sulfoxide, i.e. rabeprazole. There are different processes disclosed in the literature performing such oxidation in a wide range of conditions to reduce the by-products and increase the yield because this last step of oxidation is very difficult to perform with good results on an industrial scale. Therefore, such oxidation is considered to be the key step of most of the processes disclosed for preparing rabeprazole. Such oxidation can be carried out using conventional oxidizing agents such as 3-chloroperbenzoic acid (also known as meta-chloroperbenzoic acid or MCPBA) (cf. Uchida et al., Chem. Pharm. Bull. 1990, vol. 38, p. 534), hypochlorite, hydrogen peroxide (with or without catalysts, cf. EP 302720-A1), N-chloro or N-bromo succinimide and the like.

As aforesaid, such oxidation entails the formation of many by-products. The PCT application WO 2006100243-A1 discloses a specific impurity obtained, N-oxide pyridine derivative, a problem repeatedly found in the oxidation reaction of pyridines.

Another commonly reported by-product in previous synthetic routes is the sulfone compound. This is generated in the oxidation step by the over-oxidation of the sulfide during the preparation of the sulfoxide. W09902521-A1 further discloses the difficulty of separating and purifying sulfoxides, since the physicochemical properties of sulfoxides and the corresponding sulfones closely resemble each other. The formation of sulfones, due to over-oxidation, compromising the final yield is almost impossible to avoid. Although a possible solution is to perform the oxidation process at low temperature and restrict the amount of oxidizing agent, this increases the reaction time and decreases the final yield and additionally some starting material, such as the sulfide, does not react and has to be removed from the reaction media.

These products are described only as impurities in J. Pharm. Biomed. Anal. 2007, vol. 43, pp.1262-1269 and J. Heterocyclic Chem. 2006, vol. 43, pp. 1447-1453. In the first document, the N-oxide of rabeprazole is obtained by oxidation of rabeprazole. In the second one, the N-oxide of rabeprazole is prepared from the corresponding N-oxide sulfide derivative using potassium hydrogencarbonate and 3-chloroperbenzoic acid as oxidizing agent. The preparation of the N-oxide of rabeprazole in the conditions described in this document shows that the N-oxide of rabeprazole is obtained with low purity and high sulfone impurity content.

The removal of the above mentioned by-products (e.g. N-oxide, sulfone and sulfide) from rabeprazole has proved to be difficult, time-consuming and costly, particularly when high performance chromatography on an industrial scale is needed. None of the modifications to the oxidation step has managed to solve the problem of the formation of impurities and the difficulty of removing them.

It is well known that proton pump inhibitors of the benzimidazole-type, including rabeprazole, are very susceptible to degradation under acidic or neutral conditions. A clear indication that the impurities related to the acidic conditions are formed is that the product takes an unwanted coloration. This problem is stated in EP 1409478-A1 and in many other publications. Therefore, in the state of the art, the oxidation step from sulfide to sulfoxide had to be carried out at controlled basic pH and the reaction mixture had to be worked-up under basic conditions too.

The teaching and drawbacks of these prior art documents focus on minimizing the amount of by-products of the oxidation in the last step and point to the need for an efficient preparation process of rabeprazole using a different approach, i.e. not carrying out the problematic oxidation reaction in the last step of the synthesis.

### SUMMARY OF THE INVENTION

The inventors have found a process for the preparation of rabeprazole that overcomes the drawbacks previously mentioned. The process is based on carrying out the oxidation of a sulfide group to a sulfoxide over a compound of formula (IIIa), which is the N-oxide sulfide of a precursor of rabeprazole, using specific oxidation conditions, the oxidation not being the last step of the process. Rabeprazole is then obtained by a selective reduction of the compound obtained in the oxidation step. The above reactions can easily be carried out on an industrial scale.

Unlike rabeprazole and other proton pump inhibitors of the benzimidazole-type which are very susceptible to degradation under acidic or neutral conditions, N-oxide pyridine derivatives (both N-oxide sulfide precursor of rabeprazole and the N-oxide sulfoxide precursor of rabeprazole obtained after the oxidation) have been found to be very stable not only at a basic pH but also at an acidic pH.

It has also been found that especially good results in the oxidation step are obtained when the oxidation is carried out over the compound of formula (IIIa) to afford the compound of formula (II) at an acidic pH (i.e. pH < 7), since the purity obtained is higher than at a neutral or basic pH. This fact is detailed in Table 1 (given below) and in the Examples. Unlike that described in the prior art, the preparation of the compounds of formula (II) and (III) are obtained with significantly higher yields and purities.

The impurities due to rabeprazole acidic degradation, which are responsible for unwanted coloration, are also avoided by performing the oxidation over compound (IIIa). Likewise, due to the stability of the compounds of formula (IIIa) and (II), not only the oxidation step but also the consequent work-up can be carried out also at an acidic pH.

On the other hand, the selective reduction step of the N-oxide derivative of formula (II) at the end of the process is also advantageous because it avoids the presence of the N-oxide impurity in rabeprazole which, as explained above, is an impurity, usually found in many processes in the prior art, difficult to remove.

Thus, according to an aspect of the present invention, a process for the preparation of rabeprazole of formula (I), or its pharmaceutically acceptable salts, or its solvates, including hydrates, 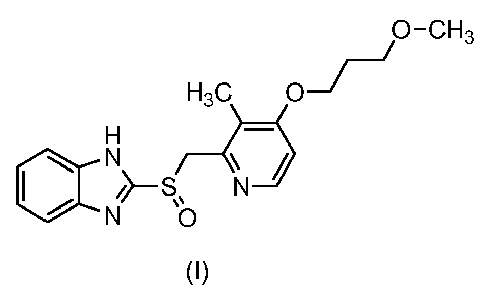 comprising reacting a compound of formula (IIIa) 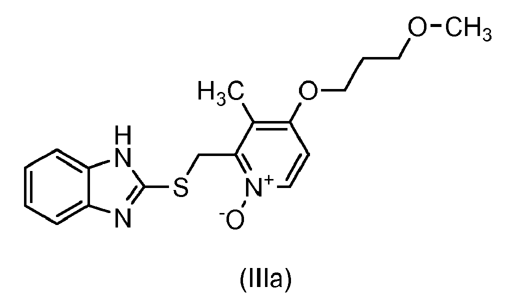 with an oxidation reagent in the presence of an acid to obtain a compound of formula (II). 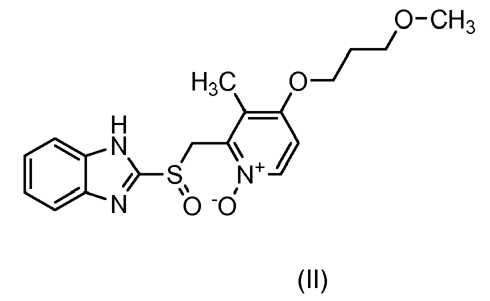 is provided.

Rabeprazole is obtained by subjecting the compound of formula (II) to a selective reduction reaction and then, if desired, treating it with a pharmaceutically acceptable base to form the corresponding salt.

Also forming part of the present invention is a process of using the compound of formula (IIIa) as intermediate in the preparation process of rabeprazole according to the first aspect of the invention, as well as a process of using the compound of formula (II) as intermediate for the preparation of rabeprazole, wherein the compound of formula (II) is selectively reduced to the compound of formula (I) and, if desired, said compound of formula (I) is reacted with a pharmaceutically acceptable base to form the corresponding salt.

The stability of compound of formula (II) in acidic media, as shown in Comparative Example 13, allows carrying out the oxidation step and the work-up step for this intermediate also in acidic media.

### DETAILED DESCRIPTION OF THE INVENTION

According to the first aspect of the invention, a wide range of oxidizing agents can be used. Useful oxidizing agents include peroxyacids, such as 3-chloroperbenzoic acid, peroxides, such as hydrogen peroxide or tert-butyl hydroperoxide, hypohalites, such as hypochlorite, N-halosuccinimides, such as N-chloro and N-bromosuccinimide, perborates, dichloroisocianurate, 1,3-dihalo-5,5-dimethylhydantoin. In a preferred embodiment, the oxidizing agent is a halogen derivative such as those described by Piotr Kowalski et al in Tetrahedron 2005, vol. 61, pp. 1933-1953. Preferably the halogen derivative is a N-halosuccinimide, more preferably N-chlorosuccinimide (NCS), or a hypochlorite, more preferably, an alkaline or alkaline-earth metal hypochlorite, such as sodium or calcium hypochlorites, or an organic hypochlorite, such as t-butyl hypochlorite.

As mentioned above, the use of acidic conditions to carry out the oxidation step has shown to be advantageous since the purity afforded is higher than in neutral or basic conditions. The acid can be an organic acid such as acetic acid or a mineral acid such as sulfuric acid or hydrochloric acid. Preferably, the acid is selected from hydrochloric acid and acetic acid. The best results are obtained when a *N*-halosuccinimide is used as oxidizing agent in the presence of an acid.

Preferably, the pH of the reaction mixture in the oxidation step is comprised between 2.5-5.5.

Preferably, the oxidation step is carried out in an appropriate solvent: Example of appropriate solvents include (C₃-C₁₀) ketones such as acetone, (C₁-C₄) alkyl (C₃-C₆) alkanoates such as ethyl acetate, acetonitrile, water and mixtures thereof. Usually, the reaction is carried out at a temperature comprised between - 40 °C and 50 °C, more preferably between - 20 and 30 °C.

Unlike other oxidizing agents, quite good results have also been obtained with *N*-halosuccinimide when the oxidation step is carried out in basic media. Thus, the use of a halosuccinimide as oxidizing agent in the presence of a base to carry out the oxidation of a compound of formula (IIIa) to afford a compound of formula (II), also forms part of the invention.

Table 1 summarizes the results afforded in the experimental part (Examples 3-10)

**Table 1**

| **Example** | **Oxidizing agent** | **pH reaction mixture** | **(%) Purity by HPLC area/area compound (II), crude/ crystallized** | **Crystallization solvent or leaching** | **% of sulfone by HPLC area/area compound (II), crude/ crystallized** |
|---|---|---|---|---|---|
| Comparative Example 3 | MCPBA | Slightly basic (aq NaHCO3) | 71.1 / 83.5 | MeOH/EtOAc | 8.71 / 3.34 |
| Example 4 | MCPBA | 3.5 - 5 | 76.2 / 88.6 | MeOH/EtOAc | 11.10 / 3.12 |
| Example 5 | NCS | Strongly basic (NaOH 50%) | 90.2 / 91.7 | MeOH/EtOAc | 0.23 / 0.07 |
| Example 6 | NCS | 3.5 - 5 | 93.7 / 97.6 | MeOH | 0.17 / 0.09 |
| Example 7 | NCS | 3.5 - 5 | 94.0 / 98.8 | MeOH/EtOAc | 0.36 / 0.11 |
| Example 8 | NCS | 3.5 - 5 | 96.6 / 98.5 | MeOH/EtOAc | 0.60 / 0.18 |
| Example 9 | Aqueous sodium hypochlorite | 5 | 88.3 / 97.4 | MeOH/EtOAc | 0.98 / 0.29 |
| Comparative Example 10 | Aqueous sodium hypochlorite | Strongly basic (NaOH 50%) | * | - | - |

| | | | | | |
|---|---|---|---|---|---|
| * In comparative Example 10, after the addition of 2 equivalents of sodium hypochlorite only 5.9 % of compound (II) was obtained, 88.2% of unreacted compound (IIIa) still remained and an unknown impurity appeared showing an area by HPLC of 3.9 % | | | | | |

The oxidation step already disclosed in the J. Heterocyclic Chem., 2006, vol. 43, p. 1447 with 3-chloroperbenzoic acid (MCPBA) under basic conditions has been reproduced in Comparative Example 3. The purity of compound of formula (II) of the crude is 71.1 % and the amount of sulfone afforded is 8.7 %. The crude is then crystallized and the sulfone impurity is reduced to 3.3 %.

These results show that a significant improvement in the purity of the compounds obtained is achieved by carrying out the oxidation step in acidic media, and that the purity is even better when a N-halosuccinimide is used as oxidizing agent.

The use of N-oxide compound of formula (IIIa) to afford the N-oxide compound of formula (II) in the oxidation, also facilitates the purification step to reduce the level of sulfone obtained by enabling it to be performed by a simple crystallization process. Surprisingly, compound of formula (II) and its corresponding sulfone are easier to purify than mixtures of rabeprazole and its corresponding sulfone, which are described in the prior art as needing several crystallization steps and therefore, as difficult to purify. Generally, the crystallization is carried out in an appropriate solvent. Examples of appropriate solvents include (C₁-C₆) alcohols such as methanol, (C₁-C₄) alkyl (C₃-C₆) alkanoates such as ethyl acetate or mixtures thereof.

The process of the invention allows a wide range of pH to be used in the work-up, since the N-oxide of rabeprazole is stable not only in basic media but also in acidic media as shown in Comparative Example 13. Therefore, unlike that which occurs with rabeprazole, the working pH of the work-up is nonrestrictive and does not need to be controlled, which greatly simplifies the purification of the N-oxide of the rabeprazole. N-oxide of rabeprazole is obtained with high yield and high purity and generally with no need for specific additional purification steps to decrease the sulfone level. However, depending on the oxidizing agent used, different amount of impurities could be formed. In order to achieve the desired purity, if needed, the pH of the work up can be adjusted to carry out a purification step in basic media or acid media because some impurities could be easier to purify at a basic pH and others at an acid pH. Furthermore, the possibility of carrying out purification at previous steps is advantageous on an industrial scale since it avoids the need for performing additional steps for the purification of the final rabeprazole.

The process of the invention includes a selective reduction step of the N-oxide derivative of formula (II) at the end of the process, as an alternative to the conventional oxidation in the last step, which avoids the presence of the N-oxide impurity in the final product. As explained above, N-oxide is an impurity usually found in many processes in the prior art. Many reducing agents have been suggested for the reduction of pyridine N-oxides into pyridines, for example, in the survey given in Houben-Weyl, "Methoden der Organischen Chemie", Vol. E7b, Part 2 (1992), 543 - 557.

In a preferred embodiment, the reducing agent used for the selective reduction mentioned above is a thiobisamine, more preferably 4,4'-thiobismorpholine.

Compound of formula (IIIa) can be prepared by reacting a compound of formula (V) with a compound of formula (IV) where R is Cl, Br, I or 3-methoxypropoxy group, in an appropriate solvent system, 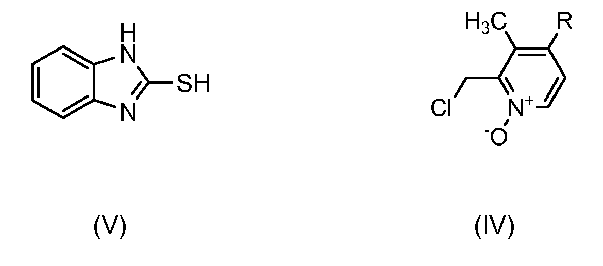 to obtain the compound of formula (III) 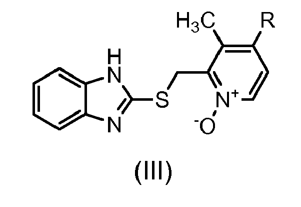 wherein R in compound (IV) and (III) is Cl, Br, I or 3-methoxypropoxy; and thereafter, if needed, transforming one R group into 3-methoxypropoxy. For instance, compound of formula (III) with R= 3-methoxypropoxy, i.e. compound of formula (IIIa) can be prepared from compound of formula (III) with R=Cl, Br or I by reaction with 3-methoxypropanol in the presence of a base and with or without solvent.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Preparation of 2-[[4-chloro-3-methyl-2-pyridinyl]-methylthio]-1H-benzimidazole N-oxide (compound of formula (IV))

1.91 g (12.71 mmol) of 2-mercaptobenzimidazole were suspended in 24 mL of methanol and treated at room temperature with a solution of 0.51 g (12.75 mmol) of NaOH in 7 mL of water. To the resultant solution were added in portions, over 5 min, 2.44 g (12.70 mmol) of 4-chloro-2-(chloromethyl)-3-methylpyridine 1-oxide synthesized following the procedure described in ES 2036948 (example 5). The reaction was stirred at room temperature over 30 min and 5 mL of water were added. The pH of the mixture was adjusted with AcOH to pH 5. The suspension formed was filtered and the solid was washed with 10 mL of water and twice with 5 mL of methanol. The solid was dried under reduced pressure at 60 °C to obtain 3.67 g (94.3%) of 2-[[4-chloro-3-methyl-2-pyridinyl]-methylthio]-1*H*-benzimidazole N-oxide.
RMN ¹H (DMSO), δ(ppm): 2.50 (s, 3H, CH₃- + DMSO); 4.80 (s, 2H, -CH₂-S); 7.10 (m, 2H, ArH); 7.34 (sa, 1H, ArH); 7.50 (d, 2H, ArH + py-H); 8.24 (d, 1H, py-H).
RMN ¹³C (DMSO), δ(ppm): 16.47, 29.21, 110.61, 117.51, 121.52, 121.90, 125.17, 131.06, 133.16, 135.86, 137.41, 143.68, 148.62, 150.48.
M.P. = 200-202 °C (decomposes).

### Example 2: Preparation of 2-[[4-[3-methoxypropoxy]-3-methyl-2-pyridinyl]-methylthio]-1H-benzimidazole N-oxide (compound of formula (III))

12.22 g (39.96 mmol) of 2-[[4-chloro-3-methyl-2-pyridinyl]-methylthio]-1H-benzimidazole N-oxide and 18 mL (188.15 mmol) of 3-methoxypropanol were heated to 120 °C. Then, 7.0 mL of NaOH 50% (132.24 mmol) were added in portions (1.0 mL/15 min). After stirring at 120 °C over 2.5 h, 250 mL of water were added and the excess of 3-methoxypropanol was removed through azeotropic distillation (T ≈ 96 °C). The residue was diluted with 40 mL of water and 160 mL of CH₂Cl₂ and the pH adjusted to 13 with NaOH conc. The phases were separated and the aqueous phase was extracted with 3 x 40 mL of CH₂Cl₂. The combined organic phases were washed with 30 mL of NaOH diluted (control pH ≈ 13), and then treated with 50 mL of water and adjusted to pH 5-6 with AcOH. The resultant organic phase was dried with anhydrous Na₂SO₄ and distilled under reduced pressure. The solid was dissolved with 30 mL of toluene and the solvent evaporated to dryness, to obtain 9.76 g (67.9%) of 2-[[4-[3-methoxypropoxy]-3-methyl-2-pyridinyl]-methylthio]-1H-benzimidazole N-oxide.
RMN ¹H (CDCl₃), δ(ppm): 2.08 (m, 2H, -CH₂); 2.33 (s, 3H, CH₃-); 3.34 (s, 3H, CH₃-O-); 3.53 (t, 2H, -CH₂-O-); 4.13 (t, 2H, -CH₂-O-); 4.76 (s, 2H, -CH₂-S-); 6.79 (d, 1H, py-H); 7.14 (m, 2H, ArH); 7.52 (m, 2H, ArH); 8.23 (d, 1H, py-H).
M.P. = 154-156 °C.

### Comparative Example 3: Preparation of 2-[[[4-[3-methoxypropoxy]-3-methyl-2-pyridinyl]-methyl]sulfinyl]-1H-benzimidazole N-oxide (compound of formula II) using 3-chloroperbenzoic as oxidizing agent in basic media

To a solution of 0.72 g (2 mmol) of 2-[[4-[3-methoxypropoxy]-3-methyl-2-pyridinyl]-methylthio]-1*H*-benzimidazole *N*-oxide in 20 mL of dichloromethane was added a solution of 0.34 g (4 mmol) of sodium hydrogencarbonate in 5 mL of water. The mixture was cooled at 0 °C and a solution of 0.44 g (1.8 mmol) of 3-chloroperbenzoic acid in 10 mL of dichloromethane was added over 2 h. The reaction mixture was stirred at the same temperature for further 2 h and then, 10 mL of water were added. After stirring for 15 min, the aqueous layer was separated and was extracted with 10 mL more of dichloromethane. The combined organic phases were treated with anhydrous Na₂SO₄ and distilled under reduced pressure to obtain 0.70 g (93%) of a crude product (purity 71.1 %, sulfone content of 8.71 % by HPLC area). The obtained solid was recrystallized from MeOH/EtOAc to obtain 0.36 g (48%) of 2-[[[4-[3-methoxypropoxy]-3-methyl-2-pyridinyl]methyl]sulfinyl]-1H-benzimidazole *N*-oxide (purity 83.5%, sulfone content of 3.34% by HPLC area).

### Example 4: Preparation of 2-[[[4-[3-methoxypropoxy]-3-methyl-2-pyridinyl]-methyl]sulfinyl]-1H-benzimidazole N-oxide (compound of formula II) using 3-chloroperbenzoic as oxidizing agent in acid media

1 g (2.78 mmol) of 2-[[4-[3-methoxypropoxy]-3-methyl-2-pyridinyl]-methylthio]-1 H-benzimidazole *N*-oxide was dissolved in 6 mL of water/acetone 1:1 mixture adjusting the pH to 5 with 1M HCl and cooled to 0-5 °C. Then, 0.68 g (2.76 mmol) of 3-chloroperbenzoic acid were added in portions over 1.5 h while adjusting the pH to between 3.5 and 5 by addition of 30% aqueous sodium hydroxide solution. The reaction mixture was stirred at the same temperature for 1.5 h maintaining the pH between 3.5 and 5. The reaction mass was extracted three times with 10 mL of dichloromethane at pH 9. The combined organic phases were dried with anhydrous Na₂SO₄ and distilled under reduced pressure to obtain 0.90 g (86%) of a solid (purity 76.2%, sulfone content of 11.1 % by HPLC area). The obtained solid was recrystallized from MeOH/EtOAc to obtain 0.60 g (58%) of 2-[[[4-[3-methoxypropoxy]-3-methyl-2-pyridinyl]methyl]sulfinyl]-1*H*-benzimidazole *N-*oxide (purity 88.6%, sulfone content of 3.12% by HPLC area).

### Example 5: Preparation of 2-[[[4-[3-methoxypropoxy]-3-methyl-2-pyridinyl]-methyl]sulfinyl]-1H-benzimidazole N-oxide (compound of formula (II)) using N-chlorosuccinimide in basic media

To a suspension of 17.1 g (47.66 mmol) of 2-[[4-[3-methoxypropoxy]-3-methyl-2-pyridinyl]-methylthio]-1*H*-benzimidazole *N*-oxide in 140 mL of water was added 7.2 mL of NaOH 50% (90 mmol) and cooled to 0 °C. Then, 9.83 g (73.62 mmol) of N-chlorosuccinimide were added in portions over 1 h. The mixture was kept at 0°C for a further 15 min and then a furher 30 min at room temperature. The reaction was treated with 4.72 g of Na₂S₂O₅ (24.8 mmol) and after stirring for 30 min, 100 mL of CH₂Cl₂ were added and the pH was adjusted to 5 with AcOH. The phases were separated and the aqueous phase was extracted twice with 70 mL of CH₂Cl₂. The combined organic phases were treated with anhydrous Na₂SO₄ and distilled under reduced pressure. The dried solid was suspended in 180 mL of methylethylketone at 50°C over 15 min and filtered at the same temperature to obtain 10.5 g of crude compound of formula (II) (purity 90.2%, sulfone content of 0.23% by HPLC area). This solid could be further purified by crystallization from MeOH/EtOAc to obtain 2-[[[4-[3-methoxypropoxy]-3-methyl-2-pyridinyl]methyl]sulfinyl]-1H-benzimidazole *N*-oxide (purity 91.7%, sulfone content of 0.07% by HPLC area).
RMN ¹H (CDCl₃), δ(ppm): 2.05 (m, 2H, -CH₂); 2.23 (s, 3H, CH₃-); 3.34 (s, 3H, CH₃-O-); 3.51 (t, 2H, -CH₂-O-); 4.09 (t, 2H, -CH₂-O-); 4.94-5.22 (dd, 2H, -CH₂-SO-); 6.76 (d, 1H, py-H); 7.31 (m, 2H, ArH); 7.69 (m, 2H, ArH); 8.21 (d, 1H, py-H).
M.P. = 155-157 °C (decomposes)

### Example 6: 2-[[[4-[3-methoxypropoxy]-3-methyl-2-pyridinyl]-methyl]sulfinyl]-1H-benzimidazole N-oxide (compound of formula II) using of N-chlorosuccinimide in acidic media

A mixture of 0.5 g (1.39 mmol) of 2-[[4-[3-methoxypropoxy]-3-methyl-2-pyridinyl]-methylthio]-1H-benzimidazole N-oxide, 2 mL of water and 1.5 mL of acetonitrile was adjusted to pH 5 with acetic acid and after cooling to 0-5 °C, 0.27 g (2.02 mmol) of N-chlorosuccinimide were added in one portion. The reaction mixture was stirred at the same temperature for 2 h while adjusting the pH to between 3.5 and 5 by addition of 30% aqueous sodium hydroxide solution. After completion, 0.13 g of Na₂S₂O₅ (0.69 mmol) were added and the solution was stirred for 30 minutes. The reaction mixture was extracted with 2.5 mL of dichloromethane twice. The combined organic phases were dried with anhydrous Na₂SO₄ and distilled under reduced pressure to obtain a crude in a quantitative yield (purity 93.7%, sulfone content of 0.17% by HPLC area). The obtained solid was recrystallized from MeOH to obtain 2-[[[4-[3-methoxypropoxy]-3-methyl-2-pyridinyl]methyl]sulfinyl]-1H-benzimidazole N-oxide (purity 97.6%, sulfone content of 0.09% by HPLC area).

### Example 7: 2-[[[4-[3-methoxypropoxy]-3-methyl-2-pyridinyl]-methyl]sulfinyl]-1H-benzimidazole N-oxide (compound of formula (II)) using of N-chlorosuccinimide in acidic media

A mixture of 2 g (5.56 mmol) of 2-[[4-[3-methoxypropoxy]-3-methyl-2-pyridinyl]-methylthio]-1H-benzimidazole N-oxide, water (9 mL) and acetone (13 mL) was adjusted to pH 4.5 with 1M HCl and cooled to 0-5 °C. Then, 1.11 g (8.31 mmol) of N-chlorosuccinimide were added in portions over 1 h while adjusting the pH to between 3.5 and 5 by addition of 30% aqueous sodium hydroxide solution. The reaction mixture was stirred at the same temperature for 2 h maintaining the pH between 3.5 and 5. After completion, 0.42 g of Na₂S₂O₅ (2.21 mmol) were added and the solution was stirred for 30 minutes. The reaction mass was extracted twice with 10 mL of dichloromethane at pH 5. The combined organic phases were dried with anhydrous Na₂SO₄ and distilled under reduced pressure to obtain a solid in a quantitative yield (purity 94.0%, sulfone content of 0.36% by HPLC area). The obtained solid was recrystallized from MeOH/EtOAc to obtain 2-[[[4-[3-methoxypropoxy]-3-methyl-2-pyridinyl]methyl]sulfinyl]-1H-benzimidazole N-oxide (purity 98.8%, sulfone content of 0.11 % by HPLC area).

### Example 8: 2-[[[4-[3-methoxypropoxy]-3-methyl-2-pyridinyl]-methyl]sulfinyl]-1H-benzimidazole N-oxide (compound of formula (II)) using of N-chlorosuccinimide in acidic media

A mixture of 12 g (33.4 mmol) of 2-[[4-[3-methoxypropoxy]-3-methyl-2-pyridinyl]-methylthio]-1H-benzimidazole N-oxide, water (36 mL) and acetone (36 mL) was adjusted to pH 5 with 2M HCl and cooled to 0-5 °C. Then, 6.7 g (50.1 mmol) of N-chlorosuccinimide were added in 0.45 g portions every 7 min. while adjusting the pH to between 3.5 and 5 by addition of 30% aqueous sodium hydroxide solution. The reaction mixture was stirred at the same temperature for 2 h maintaining the pH between 3.5 and 5. After completion, 3.1 g of Na₂S₂O₅ (16.3 mmol) were added and the solution was stirred for 30 minutes at 20-25°C. The acetone was removed by vacuum distillation and a suspension was obtained. The mixture was adjusted to pH 5. After stirring at 0-5°C for 30 min., the mixture was filtered and the solid washed with water. The solid was dried at 40°C in vacuum to obtain 10.65 g (85%) of crude 2-[[[4-[3-methoxypropoxy]-3-methyl-2-pyridinyl]-methyl]sulfinyl]-1H-benzimidazole N-oxide (purity 96.6%, sulfone content of 0.60% by HPLC area). 10.52 g of the obtained solid were purified by treating it with 52 mL of MeOH at 50°C for 45 min. After 15 min at 20-25°C, 78 mL of AcOEt were added and the suspension was left stirring for 1 hour. The mixture was further cooled to 0-5°C for 10 min. and the solid filtered and dried under vacuum at 40°C to obtain 8.15 g (78%) of purified 2-[[[4-[3-methoxypropoxy]-3-methyl-2-pyridinyl]methyl]sulfinyl]-1H-benzimidazole N-oxide (purity 98.5%, sulfone content of 0.18% by HPLC area).

### Example 9: Preparation of 2-[[[4-[3-methoxypropoxy]-3-methyl-2-pyridinyl]-methyl]sulfinyl]-1H-benzimidazole N-oxide (compound of formula (II)) using of sodium hypochlorite in acidic media

0.93 g (2.6 mmol) of 2-[[4-[3-methoxypropoxy]-3-methyl-2-pyridinyl]-methylthio]-1H-benzimidazole N-oxide were dissolved in a mixture of 2.8 mL of water and 2.8 mL of acetone. The solution was cooled at 0-5°C and adjusted to pH 5 with the addition of 2N HCl. To the resultant mixture 1.9 mL (2.5 mmol) of an aqueous solution of 10% sodium hypochlorite were slowly added while keeping the pH at 5 with addition of 2N HCl. After stirring for two hours at the same temperature, 1.9 mL (2.5 mmol) more of aqueous solution of 10% sodium hypochlorite were added, also keeping the pH at 5 with addition of 2N HCl. After 2 hours, the mixture was adjusted to pH 9 and extracted twice with 10 mL of CH₂Cl₂. The combined organic phases were dried with anhydrous Na₂SO₄ and distilled under reduced pressure to obtain 0.90 g of a crude solid (purity 88.3%, sulfone content of 0.98% by HPLC area). 0.85 g of the obtained solid was recrystallized from MeOH/EtOAc to obtain 0.46 g (54%) of 2-[[[4-[3-methoxypropoxy]-3-methyl-2-pyridinyl]methyl]sulfinyl]-1H-benzimidazole N-oxide (purity 97.4%, sulfone content of 0.29% by HPLC area).

### Example 10: Preparation of 2-[[[4-[3-methoxypropoxy]-3-methyl-2-pyridinyl]-methyl]sulfinyl]-1H-benzimidazole N-oxide (compound of formula (II)) using of sodium hypochlorite in basic media

1.0 g (2.8 mmol) of 2-[[4-[3-methoxypropoxy]-3-methyl-2-pyridinyl]-methylthio]-1H-benzimidazole N-oxide was suspended in a mixture of 3.5 mL of water and 3.5 mL of acetone. To the resultant mixture 30% aqueous sodium hydroxide solution was added until the solid was dissolved (pH 13). The mixture was cooled at 0-5 °C and 2.1 mL (2.8 mmol) of an aqueous solution of 10% sodium hypochlorite were slowly added. After stirring for two hours at 0-5°C only a small amount of sulfoxide product was observed by TLC. At that point, 2.1 mL (2.8 mmol) more of an aqueous solution of 10% sodium hypochlorite were slowly added and the reaction was left stirring for 3 hours. The mixture was adjusted at pH 10 with the addition of 2M HCl and extracted twice with 6 mL of CH₂Cl₂. The combined organic phases were dried with anhydrous Na₂SO₄ and distilled under reduced pressure to obtain a crude solid wherein the starting material shows a 88.2 % area by HPLC and only 5.9% of the area corresponds to the desired oxidation product, 2-[[[4-[3-methoxypropoxy]-3-methyl-2-pyridinyl]methyl]sulfinyl]-1H-benzimidazole N-oxide.

### Example 11: Preparation of 2-[[[4-[3-methoxypropoxy]-3-methyl-2-pyridinyl]-methyl]sulfinyl]-1H-benzimidazole (compound of formula (I), rabeprazole)

To a suspension of 0.45 g (1.20 mmol) of 2-[[[4-[3-methoxypropoxy]-3-methyl-2-pyridinyl]-methyl]sulfinyl]-1H-benzimidazole N-oxide and 0.32 g (1.57 mmol) of 4,4'-thiobismorpholine (synthesized as described by J. L. Kice et al. , J. Org. Chem. 56 (1991) 5235-6) in 8.5 mL of methanol was added dropwise a solution of 0.27 g (2.49 mmol) of chlorotrimethylsilane in 0.9 mL of ethanol. After stirring the reaction over 1.5 h at 0°C, a solution of 0.10 g (2.50 mmol) of NaOH in 2.2 mL of water was added. The alcohol solvents were removed by distillation at reduced pressure and the residue was dissolved with 12 mL of water and adjusted to pH 13 with 50% NaOH. The resultant aqueous phase was washed with 8 mL of CH₂Cl₂ three times, the pH was adjusted to pH 9-10 with aqueous solution of ammonium acetate and extracted three times with 10 mL of CH₂Cl₂. The combined organic phases were washed twice with aqueous solution of NaHCO₃ at pH 9, dried with anhydrous Na₂SO₄ and evaporated to dryness to obtain 0.34 g (79%) of 2-[[[4-[3-methoxypropoxy]-3-methyl-2-pyridinyl]-methyl]sulfinyl]-1H-benzimidazole . This solid could be further purified by crystallizing from CH2Cl2/triethylamine/heptane.
RMN ¹H (DMSO), δ(ppm): 1.96 (m, 2H, -CH₂); 2.12 (s, 3H, CH₃-); 3.23 (s, 3H, CH₃-O-); 3.46 (t, 2H, -CH₂-O-); 4.09 (t, 2H, -CH₂-O-); 4.66-4.81 (dd, 2H, -CH₂-SO-); 6.94 (d, 1H, py-H); 7.29 (m, 2H, ArH); 7.64 (m, 2H, ArH); 8.20 (d, 1H, py-H).

### Example 12: Preparation of 2-[[[4-[3-methoxypropoxy]-3-methyl-2-pyridinyl]-methyl]sulfinyl]-1H-benzimidazole sodium salt (rabeprazole sodium salt)

To 0.34 g (0.95 mmol) of 2-[[[4-[3-methoxypropoxy]-3-methyl-2-pyridinyl]-methyl]sulfinyl]-1H-benzimidazole was added a solution of 0.038 g (0.95 mmol) of NaOH in 9.2 mL of water. The mixture was stirred at room temperature until a clear solution was obtained. Then, ethanol was added to remove the water azeotropically. Finally, diethyl ether was added to the residue, filtrated and dried under reduced pressure at 75 °C affording 0.25 g (69.4 %) of a solid of 2-[[[4-[3-methoxypropoxy]-3-methyl-2-pyridinyl]-methyl]sulfinyl]-1H-benzimidazole sodium salt.
RMN ¹H (DMSO), δ(ppm): 1.97 (m, 2H, -CH₂); 2.16 (s, 3H, CH₃-); 3.24 (s, 3H, CH₃-O-); 3.48 (t, 2H, -CH₂-O-); 4.09 (t, 2H, -CH2-O-); 4.39-4.71 (dd, 2H, -CH₂-SO-); 6.87 (m, 2H, ArH); 6.93 (d, 1H, py-H); 7.46 (m, 2H, ArH); 8.28 (d, 1H, py-H).
RMN ¹³C (DMSO), δ(ppm): 11.03, 28.90, 58.18, 60.16, 65.18, 68.53, 106.18, 117.53, 118.45, 121.96, 146.84, 148.18, 152.52, 162.58, 162.86.
M.P. = 140-142 °C (decomposes).

### Comparative Example 13: Stability of 2-[[[4-[3-methoxypropoxy]-3-methyl-2-pyridinyl]-methyl]sulfinyl]-1H-benzimidazole N-oxide vs 2-[[[4-[3-methoxypropoxy]-3-methyl-2-pyridinyl]-methyl]sulfinyl]-1H-benzimidazole in acidic medium

11.7 mg of 2-[[[4-[3-methoxypropoxy]-3-methyl-2-pyridinyl]-methyl]sulfinyl]-1 H-benzimidazole N-oxide were treated with 2 mL of an aqueous acetic acid solution (pH is 4). In parallel, 11.7 mg of 2-[[[4-[3-methoxypropoxy]-3-methyl-2-pyridinyl]-methyl]sulfinyl]-1H-benzimidazole sodium salt were also treated with 2 mL of the same aqueous solution. Both mixtures were stirred at room temperature and their stability checked by TLC (CH₂Cl₂/MeOH 9:1). After 1 h, the solution of 2-[[[4-[3-methoxypropoxy]-3-methyl-2-pyridinyl]-methyl]sulfinyl]-1 H-benzimidazole N-oxide remains unaffected while the solution of 2-[[[4-[3-methoxypropoxy]-3-methyl-2-pyridinyl]-methyl]sulfinyl]-1H-benzimidazole turns dark and shows two degradation impurities with higher intensity than the starting material by TLC.

## Claims

1. A process for the preparation of rabeprazole of formula (I), or a pharmaceutically acceptable salt thereof, or a solvate thereof, including a hydrate, 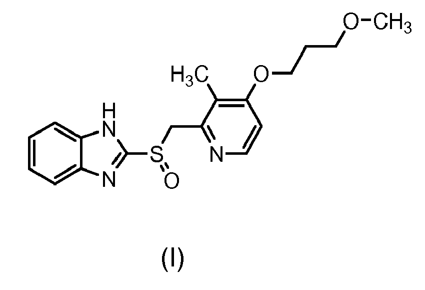 comprising reacting a compound of formula (IIIa) 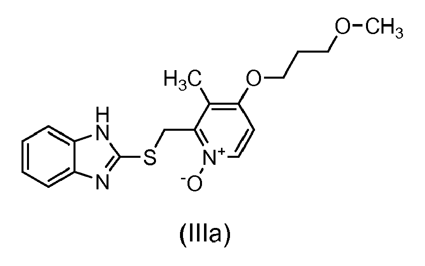 with an oxidizing agent in the presence of an acid to obtain a compound of formula (II).

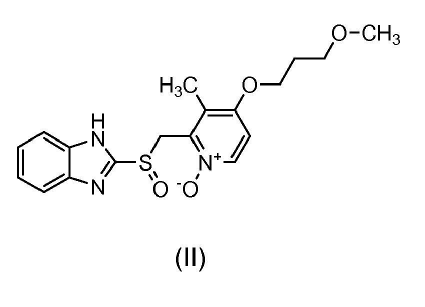

2. The process according to claim 1, further comprising submitting the compound of formula (II) to a selective reduction reaction to afford the compound of formula (I) and, if desired, treating said compound of formula (I) with a pharmaceutically acceptable base to form the corresponding salt.

3. The process according to any of the claims 1-2, wherein the oxidation step is performed with a N-halosuccinimide or a hypochlorite as oxidizing agent.

4. The process according to claim 3, wherein the N-halosuccinimide is N-chlorosuccinimide or sodium hypochlorite.

5. The process according to any of the claims 1-4, wherein the acid is selected from hydrochloric acid and acetic acid.

6. The process according to any of the claims 1-5, wherein the reduction step is performed with thiobisamine as reducing agent.

7. The process according to claim 6, wherein the thiobisamine is 4,4'-thiobismorpholine.

8. The process according to any of the claims 1-7, further comprising the reaction between a compound of formula (V) 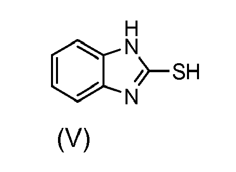 and a compound of formula (IV) 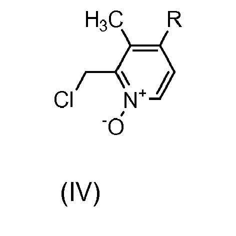 in an appropriate solvent system, to obtain the compound of formula (III) 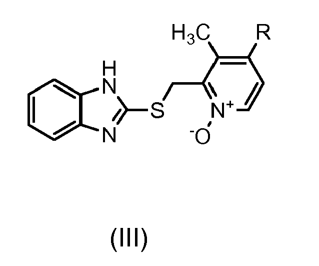 wherein R in compound (IV) and (III) is selected from the group consisting of Cl, Br, I and 3-methoxypropoxy group; and thereafter, if needed, transforming the compound obtained into a compound of formula (III) with R= 3-methoxypropoxy

9. A process of using the compound of formula (IIIa) as intermediate in the preparation process of rabeprazole of any of the claims 1-8.

10. A process of using the compound of formula (II) as intermediate for the preparation of rabeprazole, wherein the compound of formula (II) is selectively reduced to the compound of formula (I) and, if desired, said compound of formula (I) is reacted with a pharmaceutically acceptable base to form the corresponding salt.
